# EUROPEAN PATENT APPLICATION

(11) **EP 4 684 725 A1**
(43) Date of publication of application: **28.01.2026**
(21) Application number: 24190383.0
(22) Date of filing: 23.07.2024
(51) Int. Cl.: A61B 5/00, A61B 5/055, A61B 6/04

(54) **PATIENT SUPPORT FOR A MEDICAL SCANNER**

(71) Applicant: Koninklijke Philips N.V., 5656 AG Eindhoven (NL)
(72) Inventor: BOERNERT, Peter Ulrich, Eindhoven (NL); NEHRKE, Kay, Eindhoven (NL)
(74) Representative: Philips Intellectual Property & Standards

(57) **Abstract**

A patient support for a medical imaging scanner is used to support a patient as they move into a scanner bore of the medical imaging scanner. A pair of lateral wings extends laterally from a central support area. The lateral wings each have an undeployed position in which they hang down from the sides of the central support area and a deployed position in which that are curled up to form a lateral barrier at the sides of the central support area. The lateral wings are configured to adopt the deployed position when the patient support moves into the scanner bore. In this way, a protection is provided for the fingers or arms of the patient as they enter (and reside in) the scanner bore.

## Description

### FIELD OF THE INVENTION

This invention relates to medical scanners and in particular relates to the patient support of a medical scanner.

### BACKGROUND OF THE INVENTION

A medical scanner typically comprises a scanner bore through which a patient is moved in order to construct an image of a 3D volume of a patient. The patient is supported on a patient table having a movable top.

Getting the patient quickly in and out of the scanner bore is an important workflow and safety aspect which is an element of the overall patient throughput optimization. During patient preparation, the operator ensures that the upper and lower patient extremities are positioned such that fingers are not at risk of being pinched when the patient is moved into the scanner bore and such that the elbows do not touch too tightly the inner magnet system wall. This prevents undesired potential friction or slight heating from the body coil operation during scanning.

The operator thus must take special care in checking or positioning the arms and hands, often applying some extra cushion padding for the elbows. Ceiling camera technology can support the operator in this task. This can be used to supervise and guide the imaging staff during patient preparation, but this adds expense to the system. Simplifications are desired to further increase patient throughput, and also to assist the implementation of patient scanner automation (for example for so-called autonomous MRI) by reducing the complexity of the patient preparation without impacting safety.

### SUMMARY OF THE INVENTION

The invention is defined by the claims.

According to examples in accordance with this disclosure, there is provided a patient support for a medical imaging scanner for supporting a patient as they move into a scanner bore of the medical imaging scanner, comprising:
a pair of lateral wings extending laterally from a central support area,
wherein the lateral wings each have an undeployed position in which they hang down from the sides of the central support area and a deployed position in which they are curled up to form a lateral barrier at the sides of the central support area,
and wherein the lateral wings are configured to adopt the deployed position when the patient support moves into the scanner bore.

This patient support provides a simple low cost solution for protecting the patient when they enter a scanner bore. The solution can also support autonomous patient positioning and scanning. The lateral wings are for example a thin semi-stiff mattress material, and the wings are mounted at both sides of the patient table. The lateral extension of the lateral wings is larger than the width of the patient table, and, in particular, in the undeployed position the overall width is greater than the width of the scanner bore, so that the lateral wings engage with the scanner bore when the patient support is moved into the scanner bore. The lateral wings fold around the lying patient while the patient is moved into the scanner bore.

The lateral wings are for example configured to move to the deployed position by engagement of the lateral wings with an entrance to the scanner bore. For example, the scanner bore has conical inlet shape, and this shape together with the lateral wing material properties, and optionally additional guidance or support structures, are chosen to ensure the desired folding behavior, to provide patient safety and comfort.

The lateral wings for example are formed of a deformable foam, plastics, rubber or composite structure.

In one example, the patient support comprises the central support area, and the central support area and the pair of lateral wings are parts of a patient mattress. Thus, the central support area and the lateral wings are part of a single mattress under the lying patient, which moves along with the patient into the magnet. The "patient support" as defined in this disclosure then sits on top of the patient table.

The central support area of the patient mattress of this example for example corresponds in size and shape to the movable top of the patient table over which the patient mattress is provided, and the patient mattress comprises a skirt all around the central support area.

In another example, the central support area comprises the movable top of a patient table, and the lateral wings are attached to the movable top of the patient table. Thus, the "patient support" of this disclosure may then only comprise the lateral wings which are added to the lateral sides of a conventional patient table.

This disclosure also provides a patient table, comprising:
a static base;
a movable top; and
the patient support as defined above.

The patient table may further comprise a support structure for lifting the lateral wings of the patient support from the undeployed position to an intermediate position, wherein the lateral wings are configured to move from the intermediate position to the deployed position by engagement of the lateral wings with an entrance to the scanner bore.

In this way, the folding of the lateral wings is controlled more fully, with less reliance on the interaction between the lateral wings and the entrance to the scanner bore.

The support structure for example comprises lateral lifting bars. The lifting bars lifts the lateral wings partially, and the lifting is completed by engagement with the scanner bore.

The lateral lifting bars are preferably coupled to the static base. Thus, they remain outside the scanner bore, and only guide the shape of the lateral wings as they enter the scanner bore.

This disclosure also provides a medical scanner comprising:
a scanner bore; and
the patient table as defined above.

This disclosure also provides a method of supporting a patient before a scan using a medical imaging scanner having a scanner bore, comprising:
supporting the patient on a patient support comprising a pair of lateral wings which extend laterally from a central support area,
positioning the lateral wings in an undeployed position in which they hang down from the sides of the central support area; and
moving the patient support into the scanner bore,
wherein the method comprises moving the lateral wings to a deployed position in which they are curled up to form a lateral barrier at the sides of the central support area, when the patient support moves into the scanner bore.

The method may comprise moving the lateral wings to the deployed position by engagement of the lateral wings with an entrance to the scanner bore. The method may further comprise lifting the lateral wings of the patient support from the undeployed position to an intermediate position before moving the patient support into the scanner bore, and moving the lateral wings from the intermediate position to the deployed position by engagement of the lateral wings with an entrance to the scanner bore.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a medical scanner;
Fig. 2 shows a first example of a mattress design, with different stages of advancement of a patient table into a scanner bore;
Fig. 3 shows a second example of a mattress design, with different stages of advancement of a patient table into a scanner bore;
Fig. 4 shows a third example of a mattress design, with different stages of advancement of a patient table into a scanner bore; and
Fig. 5 shows a display that may be used to assist a patient in correct positioning.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a patient support for a medical imaging scanner that is used to support a patient as they move into a scanner bore of the medical imaging scanner. A pair of lateral wings extends laterally from a central support area. The lateral wings each have an undeployed position in which they hang down from the sides of the central support area and a deployed position in which that are curled up to form a lateral barrier at the sides of the central support area. The lateral wings are configured to adopt the deployed position when the patient support moves into the scanner bore. In this way, a protection is provided for the fingers or arms of the patient as they enter (and reside in) the scanner bore.

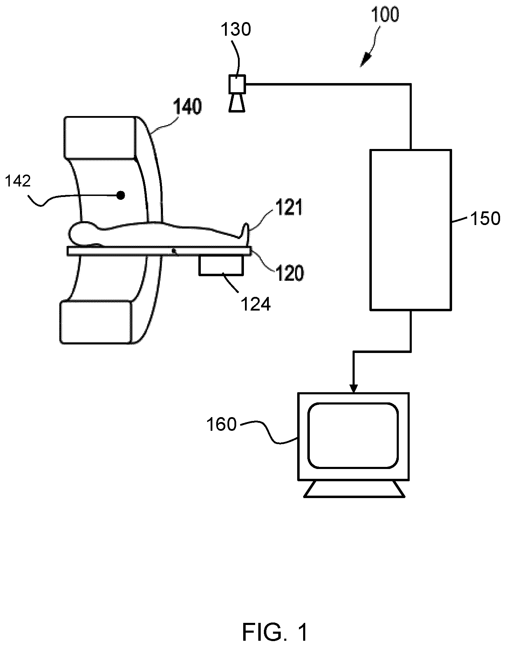

Fig. 1 shows schematically an example of a medical scanner 100 for acquiring a medical image of a subject.

The medical scanner 100 comprises a scanning system such as a CT or MRI imaging system 140 that is adapted to acquire a medical image of a patient 121 positioned on a patient table 120. The imaging system 140 has a scanner bore 142. The patient support 120 is adapted to move the patient 121 through the imaging system 140 during the imaging procedure. For this purpose, the medical scanner has a drive system 124 for driving a movable top part of the patient table 120 through the scanning system.

Fig. 1 also shows a monitoring system in the form of an optical camera 130 that is adapted to acquire monitoring images of the patient 121 during the imaging procedure. The monitoring system enables the positions of the extremities of the patient to be monitored.

A processor 150 performs the processing of the imaging data. The processor 150 generates images for display on a monitor 160.

This disclosure relates to a design of the patient support which assists in ensuring the correct position of the patient for the scanning procedure. In particular, this disclosure provides lateral wings on the two opposite sides of a central support area of the patient table, or arranged as a single mattress with lateral wings that is placed over the patient table and under the lying patient.

Both options are described as "a mattress" below and in both cases the width of the mattress is larger than the width of the patient table. The mattress moves along with the patient and the top movable part of the patient table into the scanner bore.

In use, the lateral wings are folded around the lying patient, forming a protective enclosure around the patient (in the form of a hull or cocoon) while the patient is moved into the scanner bore.

The folding of the lateral wings around the patient takes place automatically when the movable top part of the patient table is moved into the scanner bore. This is achieved by the interaction between the lateral wings and the entrance to the scanner bore, and in particular the conical inlet shape of the scanner bore combined with the mattress material properties. Thus, an automatic folding behavior is enabled. The patient table and/or the mattress may also have additional features relating to the support of the mattress over the patient table which also enable this automatic folding functionality to provide the desired patient protection.

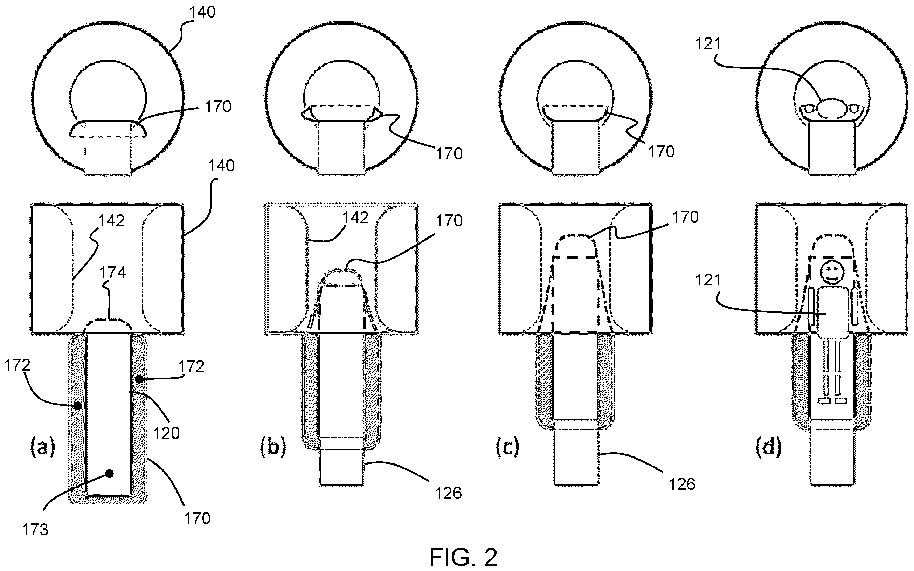

Fig. 2 shows a first example of a mattress design. Figs. 2(a) to 2(d) show different stages of advancement of the patient table 120 and hence the mattress of this disclosure into the scanner bore 142. The top images show a front end view of the scanner bore (i.e. viewing in the head-to-foot direction of the patient), patient table and mattress of this disclosure, and the bottom images show a view from above. The scanner front end views show the region of entry of the patient table into the scanner bore. They clearly show the different stages of the shape of the lateral wings, but only schematically.

The patient table has a movable top part and a static base part 126. As shown in Fig. 2(a), the movable top part of the patient table 120 is surrounded by a skirt 170. The skirt at least has lateral wings 172, but in the example shown it also surrounds the top (head end) and bottom (foot end) of the patient table.

The top of the patient table may be considered to be a central support area, namely it is the solid base which supports the patient. The lateral wings 172 extends laterally outwardly from this central support area 173. In the example of Fig. 2, there is a mattress which includes the skirt and a region over the central support area, i.e., the mattress is on top of the patient table as well as extending at least laterally beyond the area of the patient table.

Before the patient table 120 enters the scanner bore 142, the lateral wings 172 hang down as can be seen in the top image of Fig. 2(a). This is an undeployed position of the lateral wings.

When the patient table begins to enter the scanner bore, as shown in Fig. 2(b), the skirt interacts with the conical entrance to the scanner bore, and as the patient table proceeds into the scanner bore as shown in Fig. 2(c), this interaction is designed to lift the skirt. The skirt then forms a lateral barrier between the patient and the scanner bore 142. The movement of the patient table provides a gentle and progressive wrapping up of the patient with the lateral wings, while moving into the scanner bore.

The lifted skirt position is a deployed position of the lateral wings.

Thus, the portion of the mattress that is outside the scanner bore hangs down, but for the portion of the mattress that is inside the scanner bore, the lateral sides are lifted around the patient, providing a lateral barrier between the patient and the scanner bore.

Fig. 2(d) shows the patient table in the same position as Fig. 2(c) but with the patient on the patient table. It shows that the mattress provides extremity (in particular arms and fingers) protection.

In the example of Fig. 2, the mattress is placed on the patient table and the patient lies on the mattress. The mattress is for example fixed to the patient table and moves along with the top of the patient table into the scanner bore. The conical bore of the scanning system has a funnel shape and mechanically guides the mattress to curl up to conform to the the curvature of the inner wall of the scanner bore.

The skirt design of Fig. 2 has a top (head end) flap 174 of the mattress that projects into the magnet bore at the initial position of the patient table, and it is designed to initiate the automatic folding function when moving into the magnet.

The patient table is typically vertically adjustable to ease the patient embarking onto the table. The flap 174 may be in the way of this patient table movement. Thus, the mattress may instead not have any portion that projects beyond the head end of the patient support.

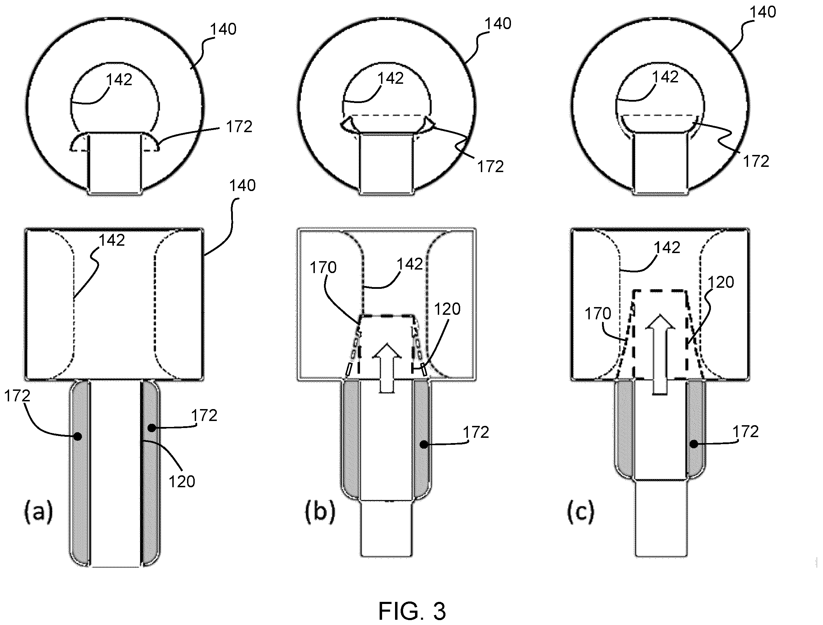

Fig. 3 shows an alternative example in which the patient support of this disclosure is formed only as the pair of lateral wings 172, and they are attached to the movable top of the patient table. Thus, the central support area is in this case the movable top of the patient table.

The lateral wings 172 do not protrude in the conical bore funnel before the patient table enters the scanner bore, and this facilitates the full vertical movement of the patient table allowing to easily pick up the patient with maximal comfort.

Figs. 3(a) to 3(c) again show different stages of advancement of the patient table 120 into the scanner bore 142. The top images again show an end view of the scanner bore, patient table and mattress, and the bottom images show a view from above.

As the patient table moves into the scanner bore, the lateral wings 172 will gently flip from their initial downward curvature to the lifted enclosing shape, protecting the patient appropriately.

The lateral wings 172 may be partly or completely stored in the patient table, when the patient table is positioned outside the scanner bore (as shown in Fig. 3(a)). When the table starts moving into the scanner bore, the lateral wings may first fold or move out horizontally from the patient table, by any suitable mechanical drive mechanism. This deployment of the lateral wings will be appropriately synchronized with the patient table movement so that the lateral wings are folded up by the further movement of the patient table into the scanner bore.

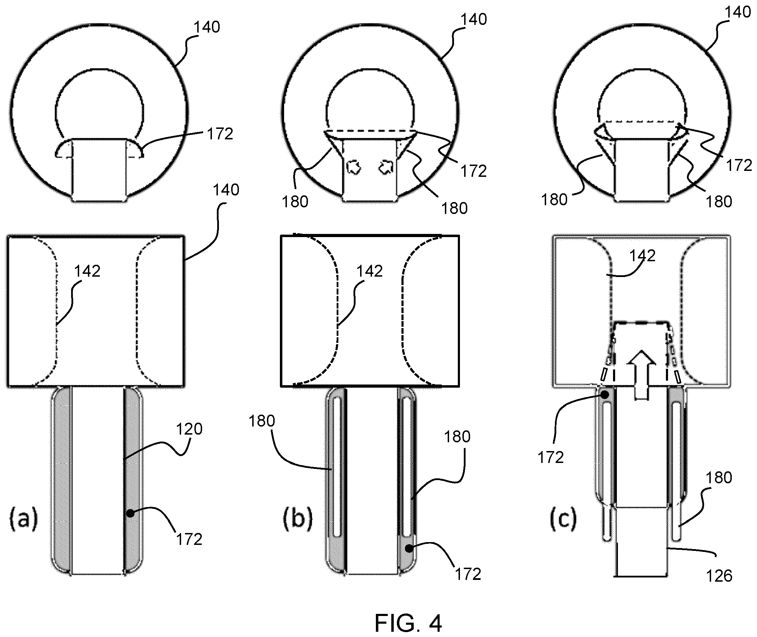

Fig. 4 shows a third example.

The mattress again comprises lateral wings 172, but additional supporting bars 180 are provided. These may for example be stored in the patient table 120, and in particular in the static base part 126 of the patient table. The supporting bars 180 are deployed and lift the lateral wings 172 even before the lateral wings interact with the scanner bore, as shown in Fig. 4(b). Thus, they function as lifting bars. They lift the lateral wings of the patient support from the undeployed position of Fig. 4(a) to an intermediate position as shown in Fig. 4(b), as can be most easily seen in the top image.

The lateral wings are configured to move from the intermediate position to the deployed position of Fig. 4(c) by the subsequent engagement of the lateral wings with an entrance to the scanner bore.

In this way, the required interaction between the mattress and the scanner bore is relaxed because the lateral wings have already been partially lifted into their final position. The supporting bars also give the mattress additional rigidity and guidance to ensure that it folds up into the desired enclosed shape (rather than crumpling). The supporting bars 180 thus guide the lateral wings into the scanner bore and support the lifting of the lateral wings. They function as guiding elements to guide the desired shape change of the mattress when it subsequently interacts with the scanner bore.

The supporting bars may be a permanent feature of the patient table, or they may be deployed only for the time the table top is moving into the scanner bore, as is the case in Fig. 4.

The supporting bars remain outside the scanner bore, as shown in Fig. 4 (c), and thus they are coupled to the static base part 126 of the patient table, rather than being mounted to the top movable part of the patient table (to which the lateral wings are coupled).

Instead of lateral supporting bars as shown, other features may be used to guide the desired movement of the lateral wings.

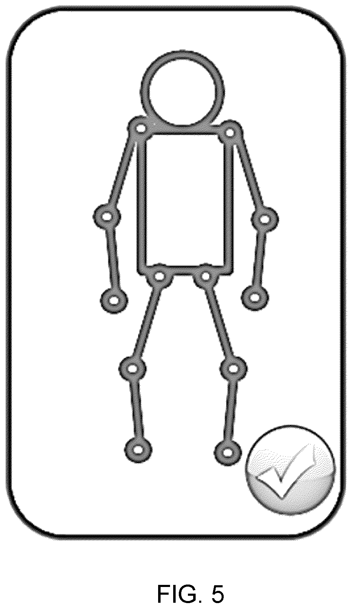

Furthermore, to support the workflow in terms of autonomous scanning, a ceiling display or poster display may be used to provide patient information, as shown in Fig. 5.

It is used to provide patient instructions to advise the patient if any of their limbs need to be moved into better positions. The patient position is for example derived from image analysis of images from the camera 130. The display does not need to convey a large amount of information and could be of low spatial resolution to ensure that people with impaired eye focus can still recognize the content. Simple patient instructions, such as via a body sketch as shown in Fig. 5, or a simple video, can be used to advise the patient of how to position themselves while moving into, and residing in, the scanner bore.

The mattress is formed of a material which has sufficient flexibility to fold up when the mattress is driven through the scanner bore, but sufficient rigidity to deformed in a predictable manner, rather than simply crumpling. Thus, it may be considered to be semi-rigid. It may be formed as a foam, plastics, rubber or composite structure. It is preferably a thin mat (e.g., less than 3 cm thick) so that it does not take up significant space of the scanner bore.

The invention may be applied to any medical scanner in which patient table is advanced into a scanner bore. Examples are a PET imaging device, an MR imaging device, a SPECT imaging device, and a CT scanner.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

Any reference signs in the claims should not be construed as limiting the scope.

## Claims

1. A patient support for a medical imaging scanner for supporting a patient as they move into a scanner bore of the medical imaging scanner, comprising:
a pair of lateral wings (172) extending laterally from a central support area (173),
wherein the lateral wings (172) each have an undeployed position in which they hang down from the sides of the central support area (173) and a deployed position in which they are curled up to form a lateral barrier at the sides of the central support area,
and wherein the lateral wings (172) are configured to adopt the deployed position when the patient support moves into the scanner bore.

2. The patient support of claim 1, wherein the lateral wings (172) are configured to move to the deployed position by engagement of the lateral wings with an entrance to the scanner bore (142).

3. The patient support of claim 1 or 2, wherein the pair of lateral wings (172) comprise a deformable foam, plastics, rubber or composite structure.

4. The patient support of any one of claims 1 to 3, wherein the patient support comprises the central support area (173), and the central support and the pair of lateral wings are parts of a patient mattress.

5. The patient support of claim 4, wherein the central support area (172) corresponds in size and shape to the movable top of a patient table over which the patient mattress is provided, and the patient mattress comprises a skirt (170) all around the central support area.

6. The patient support of any one of claims 1 to 3, wherein the central support area (173) comprises the movable top of a patient table, and the lateral wings (172) are attached to the movable top of the patient table.

7. A patient table, comprising:
a static base (126);
a movable top; and
the patient support of any one of claims 1 to 6.

8. The patient table of claim 7, further comprising a support structure (180) for lifting the lateral wings (172) of the patient support from the undeployed position to an intermediate position, wherein the lateral wings (172) are configured to move from the intermediate position to the deployed position by engagement of the lateral wings (172) with an entrance to the scanner bore (142).

9. The patient table of claim 8, wherein the support structure (180) comprises lateral lifting bars.

10. The patient table of claim 9, wherein the lateral lifting bars are coupled to the static base (126).

11. A medical scanner comprising:
a scanner bore (140); and
the patient table of any one of claims 7 to 10.

12. A method of supporting a patient before a scan using a medical imaging scanner having a scanner bore, comprising:
supporting the patient on a patient support comprising a pair of lateral wings which extend laterally from a central support area,
positioning the lateral wings in an undeployed position in which they hang down from the sides of the central support area; and
moving the patient support into the scanner bore,
wherein the method comprises moving the lateral wings to a deployed position in which they are curled up to form a lateral barrier at the sides of the central support area, when the patient support moves into the scanner bore.

13. The method of claim 12, comprising moving the lateral wings to the deployed position by engagement of the lateral wings with an entrance to the scanner bore.

14. The method of claim 12 or 13, further comprising lifting the lateral wings of the patient support from the undeployed position to an intermediate position before moving the patient support into the scanner bore, and moving the lateral wings from the intermediate position to the deployed position by engagement of the lateral wings with an entrance to the scanner bore.
